# EUROPEAN PATENT SPECIFICATION

(11) **EP 3 407 856 B1**
(45) Date of publication and mention of the grant of the patent: **04.08.2021**
(21) Application number: 16812980.7
(22) Date of filing: 22.12.2016
(51) Int. Cl.: A61K 8/34, A61K 8/36, A61K 8/368, A61Q 19/00, A61K 31/19, A61K 31/192, A61K 31/047, A61K 31/60, A61P 31/04, A61P 31/10

(54) **ACTIVE COMBINATIONS OF PERILLIC ACID AND ACTIVITY ENHANCING SUBSTANCES**
WIRKSTOFFKOMBINATIONEN VON PERILLASÄURE UND AKTIVITÄTSVERSTÄRKENDE SUBSTANZEN
COMBINAISONS ACTIVES D'ACIDE PÉRILLIQUE ET DE SUBSTANCES STIMULANT L'ACTIVITÉ

(30) Priority: 29.01.2016 EP 16153499
(43) Date of publication of application: 05.12.2018
(73) Proprietor: BRAIN Biotech AG, 64673 Zwingenberg (DE)
(72) Inventor: REHDORF, Jessica, 64625 Bensheim (DE); KLEBER, Alice, 64625 Bensheim (DE)
(74) Representative: Fuchs Patentanwälte Partnerschaft mbB
(86) International application number: PCT/EP2016/082413
(87) International publication number: WO 2017/129338

(56) References cited:
- EP-A1- 2 774 481
- DE-A1- 10 335 634
- US-A- 5 414 019

## Description

The present invention relates to compositions of perillic acid compounds and activity enhancing substances, therapeutic and non-therapeutic uses of the compositions as well as a method of preparing the compositions.

### Prior Art

Perillic acid and some derivatives are known from the art.

Perillic acid can be produced by conversion of limonene using a solvent-tolerant bacterial strain of *Pseudomonas putida.* An example for such a production process is given in Speelmans G et al., Appl Microbiol Biotechnol (1998) 50: 538-544.

DE 103 08 278 A1 describes perillic acid and its application as an active ingredient against microorganisms, including bacteria, yeasts and fungi. The use of perillic acid as preservative is described as well. Specific compositions or useful salts of perillic acid are not disclosed.

US 2010/0305214 A1 describes the use of perillic acid to increase tissue repair and decrease inflammation in tissue. The examples relate to perillic alcohol and limonene only. Specific compositions are not described.

Perillic acid has been reported to induce apoptosis in multiple myeloma cells (Beaupre DM et al., Leukemia & Lymphoma, Vol. 44, No. 12, (December 2003), pp. 2123-2134). The perillic acid composition is provided at a concentration of 20 mM in RPMI 1640 media, which is bicarbonate buffered providing a pH of above 7. Other pharmacological uses have been reported as well.

Perillic acid is known to be active against different microbial strains, especially against Gram-positive bacteria, yeasts and fungi (moulds). However, Gram-negative bacteria are less well targeted by perillic acid. It was known from DE 103 35 634 B4 that perillic acid could in principle be combined with phenoxyethanol, which is a common preservative known to be especially effective against Gram-negative bacteria. However, there is no synergistic and activity enhancing effect that goes beyond the effect expected due to the known action of both compounds. Relatively high concentrations of perillic acid and of phenoxyethanol have to be used in order to achieve reasonable effects.

A problem to be solved by the present invention was therefore to provide compositions that show broadband activity against Gram-positive and Gram-negative bacteria as well as yeasts and moulds and that are effective even at very low concentrations.

This problem is solved by the claimed subject-matter as described below.

### Description of Invention

### Compositions

In an aspect, the present invention relates to a composition comprising
- at least one perillic acid compound, and
- at least one activity enhancing substance.

The perillic acid compound is preferably selected from the group consisting of perillic acid, salts of perillic acid, hydrolysable esters of perillic acid, hydrolysable ethers of perillic acid, and/or derivatives thereof. In a preferred embodiment, the perillic acid compound is selected from perillic acid and its salts, in particular its alkali metal salts. A preferred perillic acid compound is perillic acid. The perillic acid compound is preferably selected from the salts of perillic acid, in particular the ammonium, alkali metal and alkaline earth metal salts. In preferred embodiments, the perillic acid compound is selected from perillic acid, sodium perillate, potassium perillate, ammonium perillate, calcium perillate, magnesium perillate and mixtures thereof. In particularly preferred embodiments the perillic acid compound is selected from perillic acid and sodium perillate.

It has been found by the present inventors that the compositions of the present invention have surprising advantageous effects in two different ways. First, the compositions of the present invention have a broadband effect against a large variety of microorganisms including both Gram-negative and Gram-positive bacteria as well as yeasts and fungi (moulds). Second, in addition, it has been found that combination of the perillic acid compound and the activity enhancing substance in the compositions of the present invention is accompanied by a surprising synergistic effect so that effective inhibition of microbial growth can be achieved at very low concentrations of both perillic acid compound and activity enhancing substance. Particularly, the concentration of perillic acid compound might be reduced by a factor of several hundred-fold up to several thousand-fold due to the presence of an activity enhancing substance in the composition. Thus, the activity of perillic acid compounds can be enhanced by the activity enhancing substances.

The perillic acid compounds also have the advantage that they serve as a buffer system in the composition, in particular when perillic acid is present in the composition together with a salt of perillic acid such as an ammonium, alkali metal and alkaline earth metal salt. In preferred embodiments, it is not necessary that any other buffer is added to the composition. Preferably, the compositions of this invention do not contain any buffer, in particular no bicarbonate buffer.

The inventors have now found that the antimicrobial efficacy of perillic acid compounds is remarkably pH dependent. If a certain amount of perillic acid compound is used, it will be most efficacious in a pH range of 2 to <7.5, even more efficacious at a pH of 3 to 7 or 4 to 7 and most efficacious at a pH of 4.5 to 6.5. A desirable pH is at least 2, more particularly at least 3, preferably at least 4 and most preferred at least 4.5. At a pH below the desired value the efficacy of the compound will strongly decrease again. If the pH is too high, the efficacy will decrease as well. Thus, the pH should be less than 7.5, preferably less than 7, more preferably less than 6.7, more preferably less than 6, and most preferably less than or equal to 6.5. Hence, if used at the most effective pH, the total amount of perillic acid compound in the compositions can be reduced.

The perillic acid compound is preferably used in the form of its R-enantiomer, its S-enantiomer or any mixture thereof, including racemic mixtures.

Preferably, the composition of the present invention comprises the perillic acid compound in an amount of at least 0.00001 % (w/v), more preferably at least 0.0001% (w/v), more preferably at least 0.001% (w/v), more preferably at least 0.01% (w/v), more preferably at least 0.1% (w/v). If the amount of perillic acid compound in the composition is too small, the desired antimicrobial effects cannot sufficiently be achieved. However, the amount of perillic acid compound in the composition should not be too high, because unwanted side effects could occur. Also from an economic point of view, the amount of perillic acid compound in the composition should not be higher as necessary for achieving sufficient antimicrobial effects. Therefore, the amount of perillic acid compound in the composition of the present invention is preferably at most 10% (w/v), more preferably at most 5% (w/v), more preferably at most 2% (w/v), more preferably at most 1% (w/v), more preferably at most 0.8% (w/v), more preferably at most 0.5% (w/v). Preferably, the composition of the present invention comprises the perillic acid compound in an amount of from 0.00001 % (w/v) to 10% (w/v), more preferably of from 0.0001 % (w/v) to 2% (w/v), even more preferably of from 0.001 % (w/v) to 0.5% (w/v).

Preferably, the amount (w/v) of activity enhancing substance in the compositions of the present invention is at least 10% of the amount of perillic acid compound in the compositions. More preferably, the amount (w/v) of activity enhancing substance in the compositions of the present invention is at least as high as the amount (w/v) of perillic acid compound in the compositions. More preferably, the amount of activity enhancing substance is at least twice as high, more preferably at least three times as high, more preferably at least four times as high, more preferably at least five times as high, even more preferably at least ten times as high as the amount of perillic acid compound in the compositions. However, the amount of activity enhancing substance should also not be too high. Preferably, the amount of activity enhancing substance in the compositions is at most 1000 times, more preferably at most 500 times, more preferably at most 100 times, more preferably at most 50 times, more preferably at most 35 times, more preferably at most 20 times as high as the amount of perillic acid compound in the compositions.

The preferred ratio of the amount (w/v) of activity enhancing substance in the compositions to the amount (w/v) of perillic acid compound in the compositions may vary depending on the activity enhancing substance. For example, when the activity enhancing substance is a 1,2-diole, the ratio of the amount of activity enhancing substance to the amount of perillic acid compound is preferably in the range of from 0.1 to 50, more preferably of from 1 to 10. When the activity enhancing substance is a carboxylic acid, the ratio of the amount of activity enhancing substance to the amount of perillic acid compound is preferably in the range of from 1 to 100, more preferably of from 5 to 50. When the activity enhancing substance is an aromatic alcohol, the ratio of the amount of activity enhancing substance to the amount of perillic acid compound is preferably in the range of from 2 to 200, more preferably of from 5 to 50.

The activity enhancing substance is an organic compound comprising at least two carbon atoms, more preferably at least three carbon atoms, more preferably at least four carbon atoms, more preferably at least five carbon atoms, more preferably at least six carbon atoms, more preferably at least seven carbon atoms, more preferably at least eight carbon atoms. However, the organic compound should not be too large. Otherwise, the solubility of the organic compound might be too low. Therefore, the organic compound comprises preferably at most 16 carbon atoms, more preferably at most 12 carbon atoms, more preferably at most 10 carbon atoms.

The activity enhancing substance comprises at least two terminal oxygen residues. Preferably, the activity enhancing substance comprises exactly two terminal oxygen residues. In alternative embodiments, the activity enhancing substance comprises more than two terminal oxygen residues. In such alternative embodiments, the activity enhancing substance comprises preferably exactly three terminal oxygen residues. In less preferred alternative embodiments, the activity enhancing substance comprises preferably six terminal oxygen residues, more preferably five terminal oxygen residues, more preferably four terminal oxygen residues.

A terminal oxygen residue according to the present invention is an oxygen residue that is covalently bound to exactly one carbon atom within the organic compound. Terminal oxygen residues might additionally also be bound to hydrogen. However, preferably the activity enhancing substance comprises at most two hydroxyl groups. The following scheme shows examples of terminal oxygen residues, wherein R represents an arbitrary group bound to the terminal oxygen residue(s) via carbon to oxygen bonds.

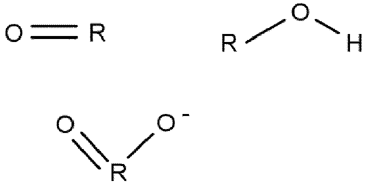

In contrast to terminal oxygen residues, oxygen residues that are covalently bound to two carbon atoms are termed bridging oxygen residues according to the present invention. The following scheme shows a bridging oxygen residues, wherein R and R' represent arbitrary groups bound to the bridging oxygen residue via carbon to oxygen bonds.

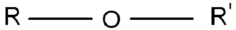

Preferably, the terminal oxygen residues are present in the activity enhancing substance in close proximity. "Being present in close proximity" means according to the present invention that the terminal oxygen residues are bound to carbon atoms that are spaced by at most two, more preferably by at most one carbon atom that is not bound to a terminal oxygen residue. More preferably, the terminal oxygen residues are bound to adjacent carbon atoms or even to the same carbon atom. Terminal oxygen residues that are bound to adjacent carbon atoms or to the same carbon atom within the activity enhancing substance form a terminal oxygen group (TO-group) together with the corresponding carbon atoms and optional hydrogen residues according to the present invention. The inventors hypothesize that the presence of a TO group is important to achieve the desired synergistic effect. Examples of TO-groups are shown in the following scheme, wherein R represents an arbitrary group bound to the TO-group via a carbon to carbon bond and R' represents an arbitrary group bound to terminal oxygen residues via carbon to oxygen bonds. Specific examples of TO groups include the vicinal diol group, the α-hydroxyl ketones and the carboxylic acid group.

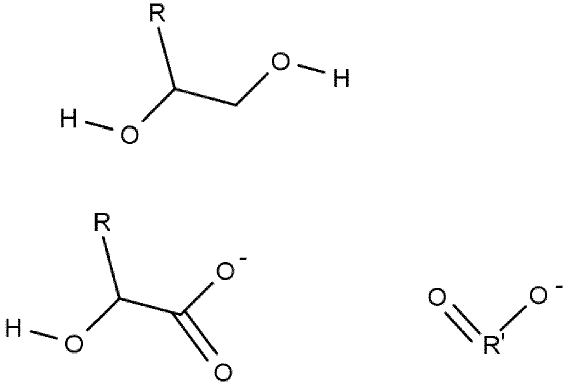

Preferably, the terminal oxygen residues are present in the activity enhancing substance in form of at least one TO-group. According to the present invention, a TO-group comprises at least two terminal oxygen residues, which are present in geminal position or in vicinal position, i.e. terminal oxygen residues bound to the same (geminal) or to adjacent (vicinal) carbon atoms within the activity enhancing substance. In alternative embodiments, the terminal oxygen residues are not present in form of TO-groups. In such alternative embodiments, the terminal oxygen residues are present in isolated form, i.e. the terminal oxygen residues are bound to carbon atoms that are not directly bound to each other.

Preferably, the TO-group is selected from the group consisting of the malic group, the glycol group and the carboxyl group as explained below. More preferably, the TO-group is selected from the group consisting of the glycol group and the carboxyl group. Preferably, the glycol group is a terminal glycol group.

The activity enhancing substance is selected from the group consisting of 1,2-diols, carboxylic acids and derivatives thereof. Preferably, the activity enhancing substance is selected from the group consisting of alkane-1,2-diols, carboxylic acids and derivatives thereof. More preferably, the activity enhancing substance is selected from the group consisting of hexane-1,2-diol, octane-1,2-diol, decane-1,2-diol, levulinic acid, p-anisic acid, propionic acid, pelargonic acid, malic acid, sodium benzoate and potassium sorbate. Even more preferably, the activity enhancing substance is selected from the group consisting of octane-1,2-diol, levulinic acid, sodium benzoate and potassium sorbate.

Preferably, the activity enhancing substance comprises exactly one TO-group. In alternative embodiments, the activity enhancing substance comprises at least two TO-groups, preferably exactly two TO-groups. Preferably, the activity enhancing substance comprises at most three TO-groups.

According to the present invention, a TO-group with terminal oxygen residues in vicinal position might comprise three terminal oxygen residues. For example, a carboxyl group (C(O)OH) might be bound to a carbon atom that is bound to a hydroxyl group. A preferred activity enhancing substance comprising such TO-group is malic acid. Therefore, such TO-group is termed "malic group" according to the present invention.

However, preferably, TO-groups comprise exactly two terminal oxygen residues according to the present invention. An especially preferred TO-group with terminal oxygen residues in vicinal position is the glycol group (C(OH)C(OH)). Especially preferably, the TO-group is a terminal glycol group, i.e. a glycol group, in which at least one of the carbon atoms is not bound to a second carbon atom. Preferably, activity enhancing substances that comprise a glycol group do not comprise any further terminal oxygen residues. Preferred activity enhancing substances comprising such TO-group are 1,2-diols. Especially preferred activity enhancing substances are alkane-1,2-diols. Preferably, the alkane-1,2-diols have at least 6 carbon atoms. Preferably, the alkane-1,2-diols have at most 12 carbon atoms. Preferably, the alkane-1,2-diols are selected from the group consisting of hexane-1,2-diol, octane-1,2-diol, decane-1,2-diol and dodecane-1,2-diol. More preferably the alkane-1,2-diol is octane-1,2-diol.

More preferred than TO-groups with terminal oxygen residues in vicinal position are TO-groups with terminal oxygen residues in geminal position. Most preferred, the TO-group with terminal oxygen residues in geminal position is a carboxyl group. Preferably, the activity enhancing substance comprises at most two carboxyl groups, more preferably at most one carboxyl group.

Preferred activity enhancing substances are selected from carboxylic acids, and derivatives thereof. According to the present invention, derivatives of carboxylic acids are selected from the group consisting of salts of carboxylic acids, hydrolysable esters of carboxylic acids, and/or hydrolysable ethers of carboxylic acids. In a preferred embodiment, the derivatives of carboxylic acids are salts thereof, in particular their alkali metal salts. Derivatives of carboxylic acids are preferably selected from the group consisting of ammonium, alkali metal and alkaline earth metal salts thereof. Sodium, potassium, ammonium, calcium and magnesium salts are more preferred. In particularly preferred embodiments the derivatives of carboxylic acids are sodium or potassium salts thereof.

Preferably, the carboxylic acids are monoacids. Preferably, the carboxylic acids have a molecular weight of more than 50 g/mol, more preferably more than 65 g/mol, more preferably more than 75 g/mol, more preferably more than 100 g/mol. Preferably, the carboxylic acids have a molecular weight of less than 250 g/mol, more preferably less than 200 g/mol, more preferably less than 160 g/mol, more preferably less than 150 g/mol.

Preferably, the carboxylic acids have a pKa of at least 2.5 more preferably at least 3.0, more preferably at least 3.5, more preferably at least 4.0. Preferably, the carboxylic acids have a pKa of at most 6.5, more preferably, at most 6.0, more preferably at most, 5.5, more preferably at most 5.0.

Preferably, the carboxylic acids are selected from the group consisting of levulinic acid, *p*-anisic acid, propionic acid, pelargonic acid, malic acid, benzoic acid, salicylic acid and sorbic acid. More preferably, the carboxylic acids are selected from the group consisting of levulinic acid, *p-anisic* acid, propionic acid, pelargonic acid, benzoic acid, salicylic acid and sorbic acid. Even more preferably, the carboxylic acids are selected from the group consisting of levulinic acid, benzoic acid, salicylic acid and sorbic acid.

The compositions of this invention preferably contain water. Microorganisms need water to grow. Thus, water containing compositions are prone to microbial spoilage. The compositions of this invention provide a way of preserving water containing compositions in an effective way. Preferred compositions of this invention are water-based. In the context of this invention, "water-based" means that water is the main ingredient in the composition, i.e. the ingredient that is present in the highest proportion when compared with the other ingredients in the composition. In preferred embodiments the amount of water in the compositions of this invention is at least 10% by weight of the composition, more preferred at least 20% by weight of the composition, more preferred at least 30% by weight, more preferred at least 40% by weight, more preferred at least 50% by weight or at least 70% by weight.

In preferred embodiments, the composition of this invention is selected from foodstuffs, food packaging, beverages, animal food, medicinal products, pharmaceutical products, cosmetic products, household products and technical products. Suitable foodstuffs include water-containing foodstuffs, in particular dairy products such as yogurt, curd, cheese, cottage cheese, grating cheese; but also marmalade, jelly. Suitable food packaging includes water-containing food packaging and food packaging that has been surface-treated with the compositions of the invention. It also includes shells, envelopes, casings, rinds and wrappings within which food can be or is packaged. Preferred embodiments include food packaging for cheese and sausage products, including cheese rinds and sausage casings. Suitable beverages include carbonated and non-carbonated beverages, in particular lemonade, beer, sparkling water, mineral water, energy drinks, milk, fruit juice, vegetable juice, smoothies and drinking yogurt, but also wine, sparkling wine, fruit wine, liquor and spirits. Suitable animal food includes water-containing animal food. Preferred embodiments include animal food in particular pet food. Suitable medical devices include medical devices of class Ila. Preferred embodiments include water-containing medicinal products, in particular wound dressings and wound cleansing products and contact lenses solutions. Suitable pharmaceutical products include water-containing pharmaceutical products. Preferred embodiments include eye drops, nose drops, aerosols, infusions, injections, solutions, emulsions, dispersions, pastes, gels, ointments, capsules, and effervescent formulations. Preferred cosmetic products include water-containing cosmetic products. Preferred embodiments include creams, lotions, ointments, deodorant sticks, pump sprays, toothpaste, mouth wash, shampoo, soap, shower gel, aerosols, sprays, solutions, emulsions, dispersions and pastes. Preferred household products include water-containing household products, in particular those used for cleaning or maintenance of water-using household appliances like washing machines, dish washers, driers, coffee machines, steam cookers etc. Preferred embodiments include detergents, washing agents and cleaning agents. Preferred technical products include water-containing technical products. Preferred embodiments include paints, lacquers, lubricants, coatings, construction materials, sealing mass, adhesives, paste and glue.

The compositions of this invention preferably comprise limonene in a concentration of less than 50 mM, preferably less than 25 mM and more preferably less than 10 mM. In preferred embodiments the compositions of this invention do not comprise any limonene in detectable amounts.

It turned out that combination of perillic acid compounds with certain other components is disadvantageous. Therefore, it is preferable that such other components are not contained in the compositions of the present invention or are contained only in minor amounts. Such components are preferably selected from the group consisting of glyceryl ethers and acetophenone derivatives such as substituted acetophenone. Preferably, the amount (w/v) of glyceryl ethers in the compositions of the present invention is at most 1%, more preferably at most 1000 ppm, more preferably at most 500 ppm, more preferably at most 100 ppm, more preferably at most 50 ppm, more preferably at most 20 ppm, more preferably at most 10 ppm, more preferably at most 1 ppm of the amount of perillic acid compound in the compositions. Preferably, the amount (w/v) of acetophenone derivatives, such as substituted acetophenone, in the compositions of the present invention is at most 1%, more preferably at most 1000 ppm, more preferably at most 500 ppm, more preferably at most 100 ppm, more preferably at most 50 ppm, more preferably at most 20 ppm, more preferably at most 10 ppm, more preferably at most 1 ppm of the amount of perillic acid compound in the compositions. More preferably, the amount (w/v) of each of glyceryl ethers and acetophenone derivatives, such as substituted acetophenone, in the compositions of the present invention is at most 1 %, more preferably at most 1000 ppm, more preferably at most 500 ppm, more preferably at most 100 ppm, more preferably at most 50 ppm, more preferably at most 20 ppm, more preferably at most 10 ppm, more preferably at most 1 ppm of the amount of perillic acid compound in the compositions. Even more preferably, the compositions of the present invention do not comprise glyceryl ethers and/or acetophenone derivatives such as substituted acetophenone.

### Uses of the compositions

The compositions of this invention are useful for a large number of applications. In an aspect of this invention, compositions as defined herein can be used for preservation, for prevention against microbial spoilage, for therapeutic treatment against microbial infection, for cosmetic care and/or treatment against microbial infection, as fungicides, as herbicides.

### Preservation

In preferred embodiments the compositions of this invention are used for preservation. This may include preservation of compositions according to this invention. Compositions that can be preserved using compositions of this invention include foodstuffs, food packaging, beverages, animal food, medicinal products, pharmaceutical products, cosmetic products, household products and technical products.

Foodstuffs that can be preserved using the compositions of this invention include water-containing foodstuffs, in particular dairy products such as yogurt, curd, cheese, cottage cheese, grating cheese; but also marmalade, and jelly. Other embodiments include preservation of sea food such as lobsters, oysters, mussels, fish and shrimps. In order to achieve the desired preserving effect the perillic acid compound and/or the compositions of this invention are preferably added to the foodstuff or applied to the foodstuff.

Food packaging that can be preserved using the compositions of this invention includes shells, envelopes, casings, rinds and wrappings within which food can be or is packaged. Preferred embodiments include food packaging for cheese and sausage products, including cheese rinds and sausage casings. In order to achieve the desired preserving effect the perillic acid compound and/or the compositions of this invention are preferably added to or applied to the food packaging.

Beverages that can be preserved using the compositions of this invention include carbonated and non-carbonated beverages, in particular lemonade, beer, sparkling water, mineral water, energy drinks, milk, fruit juice, vegetable juice, smoothies and drinking yogurt, but also wine, sparkling wine, fruit wine, liquor and spirits. In order to achieve the desired preserving effect the perillic acid compound and/or the compositions of this invention are preferably added to the beverage.

Animal food that can be preserved using the compositions of this invention include water-containing animal food. Preferred embodiments include animal food in particular pet food. In order to achieve the desired preserving effect the perillic acid compound and/or the compositions of this invention are preferably added to the animal food.

Medical devices that can be preserved using the compositions of this invention include medical devices of class Ila. Preferred embodiments include water-containing medicinal products, in particular wound dressings and wound cleansing products and contact lenses solutions. In order to achieve the desired preserving effect the perillic acid compound and/or the compositions of this invention are preferably added to or applied to the medicinal product.

Pharmaceutical products that can be preserved using the compositions of this invention include water-containing pharmaceutical products. Preferred embodiments include eye drops, nose drops, aerosols, infusions, injections, solutions, emulsions, dispersions, pastes, gels, ointments, capsules, and effervescent formulations. In order to achieve the desired preserving effect the perillic acid compound and/or the compositions of this invention are preferably added to the pharmaceutical product.

Cosmetic products that can be preserved using the compositions of this invention include water-containing cosmetic products. Preferred embodiments include creams, lotions, ointments, toothpaste, deodorant sticks, pump sprays, mouth wash, shampoo, soap, shower gel, aerosols, sprays, solutions, emulsions, dispersions and pastes. In order to achieve the desired preserving effect the perillic acid compound and/or the compositions of this invention are preferably added to the cosmetic composition.

Household products that can be preserved using the compositions of this invention include water-containing household products, in particular those used for cleaning or maintenance of water-using household appliances like washing machines, dish washers, driers, coffee machines, steam cookers etc. Preferred embodiments include detergents, washing agents and cleaning agents. In order to achieve the desired preserving effect the perillic acid compound and/or the compositions of this invention are preferably added to the household product.

Technical products that can be preserved using the compositions of this invention include water-containing technical products. Preferred embodiments include paints, lacquers, lubricants, coatings, construction materials, sealing mass, adhesives, paste and glue. In order to achieve the desired preserving effect the perillic acid compound and/or the compositions of this invention are preferably added to the technical product.

The compositions of this invention are particularly useful for preservation of water-containing products. Preferably, the water content in the compositions, wherein the perillic acid compound or composition is used for purposes of preservation is at least 10% by weight, preferably at least 20% by weight, more preferred at least 30% by weight, more preferred at least 40% by weight, more preferred at least 50% by weight, more preferred at least 60% by weight and most preferred at least 75% by weight.

In preferred embodiments the compositions of this invention are used as preservatives in emulsions. Emulsions are difficult media with regard to preservation because they comprise a lipophilic and a hydrophilic phase. Many preservatives are lipophilic and thereby accumulate in the oil phase of an emulsion. However, the phase that is most prone to bacterial spoilage is the aqueous or hydrophilic phase. The inventors have found that due to the adjustment of the pH to the desired range the preservation efficacy of the inventive preservative is maximized in emulsions.

### Antimicrobial uses

The compositions of this invention can be used as antimicrobial agents and compositions. It has been shown that the compositions of this invention are active against bacteria, yeast and moulds. It is hence a preferred embodiment of this invention to use the compositions as antimicrobial agents. Antimicrobial use includes therapeutic use and non-therapeutic use.

### Therapeutic use

In an embodiment, the compositions are used as an antimicrobial agent in a therapeutic method. The method preferably includes the step of administering to a subject an effective amount of the composition of this invention. Administration can be topical, local and/or systemic. The subject can be human or non-human. Preferred subjects are mammals, in particular humans.

Pathological conditions that can be treated with the compositions of this invention include mycotic infections, bacterial infections and inflammation. Preferred pathological conditions include athlete's foot, nail fungus, eczema, and caries.

Therapeutic use includes the antimicrobial application against nosocomial infections, including those caused by MRSA, MRSE, ESBL and VRE. In another embodiment the use of the compounds and compositions against nosocomial infections, including those caused by MRSA, MRSE, ESBL and VRE, is not therapeutic but includes application as cleansers or disinfectants.

### Non-Therapeutic use

In an embodiment, the compositions are used as an antimicrobial agent in a non-therapeutic method. The method preferably includes the step of administering to a subject or applying to an object an effective amount of the composition of this invention. Administration to a subject can be topical, local and/or systemic. Application to an object can be superficial, by admixture, coating, immersion or impregnation.

The subject can be human or non-human. Preferred subjects are mammals, in particular humans. The compounds and compositions of this invention can be used for improvement of skin balance and sebum control. Non-pathological conditions that can be treated with the perillic acid compounds and compositions of this invention include cosmetic conditions like halitosis, dandruff, rosacea, impure and large pore skin, body odor, couperose and acne.

The non-therapeutic use includes application of the composition to an object. The object can be furniture, wood, stone, metal, construction material, surfaces in cars, factories and houses, filters in air conditioners and others.

The compositions of this invention can also be used as herbicides and fungicides, in particular in plant protection products.

The compositions of this invention can be used as disinfectants in hospitals, restaurants, hotels, laundries, households, industry, animal farming etc.

### Method

The invention also includes a method of preparing a composition according to this invention including the steps of preparing a mixture of a perillic acid compound and an activity enhancing substance.

### Examples

### Example 1: Determination of minimal inhibition concentration (MIC)

The antimicrobial activity of different substances was examined and compared to perillic acid *in vitro* using the microdilution method adapted from DIN EN ISO 20776-1:2006. Besides perillic acid and its sodium salt, other organic acids like levulinic acid, p-anisic acid, benzoic acid and sorbic acid as well as alkane-1,2-diols such as hexane-1,2-diol, octane-1,2-diol and decane-1,2-diol were studied. Additionally, the commonly used preservatives 2-phenoxyethanol, 2-phenylethanol and benzyl alcohol were investigated.

All experiments were done in 96-well plates in a final volume of 200µL. Test solutions of the investigated compounds were prepared in 25mM buffered water. The procedure was conducted as following: 4µL of the particular stock solution were mixed with 196µL culture medium containing a defined inoculum of the respective target strain. Sterile controls (not inoculated) and growth controls (buffer instead of compound added) were performed. All compound concentrations were tested in triplicate for several times. The following contamination germs were examined: *Escherichia coli ATCC* 8739, *Pseudomonas aeruginosa* ATCC 9027, *Stapyhlococcus aureus* ATCC®6538, *Bacillus subtilis* subsp. *spizizenii* ATCC®6633, *Candida albicans* ATCC®10231 and *Aspergillus brasiliensis* ATCC®16404. The strains were cultivated either in Mueller-Hinton II Bouillon (*Escherichia coli, Pseudomonas aeruginosa, Staphylococcus aureus*), in yeast peptone dextrose (*Bacillus subtilis, Candida albicans*) or in potato dextrose medium (*Aspergillus brasiliensis*)*.* All culture media were buffered with 25mM MES-buffer. For compound testing, the plates were incubated at 37°C for *Escherichia coli, Pseudomonas aeruginosa* and *Staphylococcus aureus,* at 28°C for *Bacillus subtilis* and *Candida albicans,* and at 25°C for *Aspergillus brasiliensis.* Plates were evaluated after 24h and 72h, respectively. The minimal inhibitory concentration (MIC) is defined as the concentration where no microbial growth was observed after 24h and 72h incubation at the specified temperature.

The results are summarized in table 1. MIC-values are given according to the following letter code. A: MIC > 1% (w/v), B: MIC = 0.5-1% (w/v), C: MIC = 0.1-0.5% (w/v), D: MIC < 0.1% (w/v).

**Table 1**

| | Microbial Contamination Germs | | | | | |
|---|---|---|---|---|---|---|
| **MIC [A to D]** | *Escherichia coli* | *Pseudomonas aeruginosa* | *Staphylococcus aureus* | *Bacillus subtilis* | *Candida albicans* | *Aspergillus brasiliensis* |
| Test Compound | | | | | | |
| *R*-Perillic acid | C | B | D | D | D | D |
| S-Perillic acid | C | B | D | D | D | D |
| Sodium perillate | C | B | D | D | D | D |
| Levulinic acid | C | C | C | B | A | C |
| *p*-Anisic acid | C | C | C | C | B | C |
| Salicylic acid | D | D | D | D | C | C |
| Sodium benzoate | B | B | B | D | C | A |
| Potassium sorbate | B | A | A | C | B | A |
| Hexane-1,2-diol | B | B | A | A | A | B |
| Octane-1,2-diol | B | C | A | C | A | C |
| Decane-1,2-diol | C | C | D | D | D | D |
| Dodecane-1,2-diol | B | D | D | D | D | D |
| 2-Phenoxyethanol | B | B | B | B | B | B |
| Dehydroacetic acid | C | C | D | D | D | D |
| 2-Phenylethanol | B | B | B | B | B | B |
| Benzyl Alcohol | B | B | B | B | B | B |

The results show that perillic acid is highly active against Gram-positive bacteria (*Staphylococcus aureus, Bacillus subtilis*), yeasts (*Candida albicans*) and moulds (*Aspergillus brasiliensis*). Against Gram-negative bacteria (*Escherichia coli* and *Pseudomonas aeruginosa*) higher concentrations need to be applied in order to achieve growth inhibition. Notably, no difference in activity was observed between R-perillic acid, S-perillic acid and sodium perillate.

### Example 2: Combination of different substances with perillic acid

Activity of perillic acid was comparably low against Gram-negative bacteria. It was thus tested whether the respective MIC could be reduced by combination of perillic acid with selected substances.

Representative combination partners were selected based on their activity profile against Gram-negative bacteria in example 1. The tested candidates represented four groups of potential combination partners. The most promising candidates were represented by levulinic acid. Levulinic acid showed higher activity against *Pseudomonas aeruginosa* (Pa) and the activity against *Escherichia coli* (Ec) was comparable to the activity of perillic acid. Also octane-1,2-diol, representing a second group of candidates, showed higher activity against *Pseudomonas aeruginosa.* However, activity against *Escherichia coli was* lower as compared to perillic acid. The aromatic mono-alcohols 2-phenoxyethanol, 2-phenylethanol and benzyl alcohol are a third group of candidates. These three substances showed comparable activity against *Pseudomonas aeruginosa* but lower activity against *Escherichia coli* as compared to perillic acid. Finally, potassium sorbate showed lower activity against both *Pseudomonas aeruginosa* and *Escherichia coli* than perillic acid and was thus representing one of the less promising combination partners.

The results of the combination experiments are summarized in table 2. Experiments were performed as described for example 1 above. A reduction factor was determined, expressing the reduction of the MIC of perillic acid that was achievable by combination with the combination partner. For example, a reduction factor of 2 means that the MIC of perillic acid was reduced by a factor of 2 by combination of perillic acid with the respective activity enhancing substance.

**Table 2**

| | Microbial Contamination Germs | |
|---|---|---|
| **Reduction factor** | *Escherichia coli* | *Pseudomonas aeruginosa* |
| Test Compound | | |
| Levulinic acid | 200 | 40 |
| Salicylic acid | 40 | 80 |
| Potassium sorbate | 200 | 40 |
| Octane-1,2-diol | 2000 | 2000 |
| 2-Phenoxyethanol | 30 | 20 |
| 2-Phenylethanol | 40 | 8 |
| Benzyl Alcohol | 20 | 5 |

The results of this experiment are highly unexpected. Even though levulinic acid represented one of the most promising combination partners and potassium sorbate represented one of the least promising combination partners based on the results of example 1, both compounds show the same activity enhancing effect against Gram-negative bacteria when combined with perillic acid. These results suggest that the results of example 1, in which substances were separately tested for their activity against Gram-negative bacteria, do not have any predictive value with regard to the activity enhancing effect when combined with perillic acid. Rather, it seems that carboxylic acids in general are suitable activity enhancing substances. Furthermore, an even more pronounced activity enhancing effect was observed with regard to octane-1,2-diol suggesting that 1,2-diols are very strong activity enhancing substances as well. In contrast, results were rather low with regard to aromatic mono-alcohols tested. It seems that presence of at least two terminal oxygen residues boosts the activity enhancing properties in comparison to substances that comprise only one terminal oxygen residue.

### Example 3: Activity enhancing effect in cosmetic formulations

As a next step the most promising combinations of perillic acid with levulinic acid and octane-1,2-diol, respectively, were tested in different cosmetic formulations. The composition of the formulations was in general as follows (ingredient amounts according to the FDA-code):

| **Ingredient** | **Amount [%]** |
|---|---|
| Water | add 100 |
| Humectant | E |
| Thickener | F |
| Oil | C |
| Emulsifier | E |
| Na-Perillate | F-G |
| Active compound | E-F |
| pH-Regulator | q.s. |

The composition of specific examples (oil-water-emulsions) comprising levulinic acid or octane-1,2-diol is shown below.

| **Ingredient** | **Amount [% w/v]** |
|---|---|
| Aqua | 80.7 |
| Glycerol | 2.0 |
| Alkyl-acrylate crosspolymer | 0.3 |
| Sucrose stearate | 2.5 |
| Ethylhexyl palmitate | 6.0 |
| Dicaprylyl ether | 2.0 |
| Cetearyl isononanoate | 3.0 |
| Xanthan gum | 0.3 |
| Arginine | 3.0 |
| Perillic acid compound | 0.1 |
| Levulinic acid | 0.1 |
| Aqua | 80.3 |
| Glycerol | 2.0 |
| Alkyl-acrylate crosspolymer | 0.3 |
| Sucrose stearate | 2.5 |
| Ethylhexyl palmitate | 6.0 |
| Dicaprylyl ether | 2.0 |
| Cetearyl isononanoate | 3.0 |
| Xanthan gum | 0.3 |
| Arginine | 3.0 |
| Perillic acid compound | 0.1 |
| Octane-1,2-diol | 0.5 |

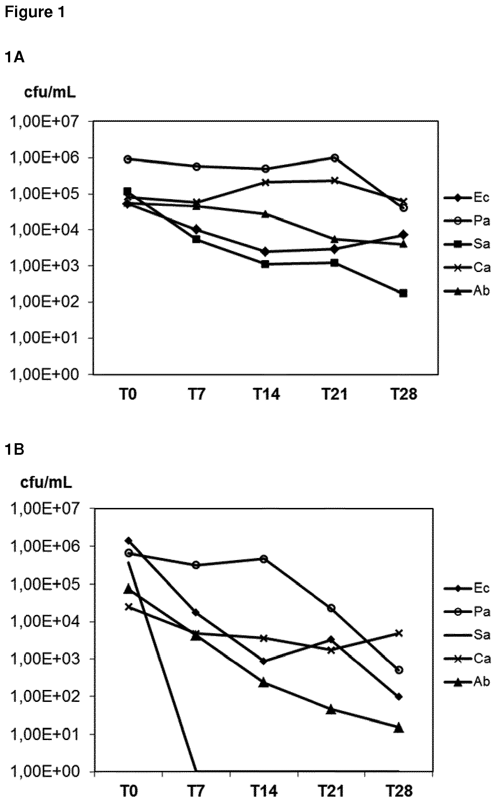

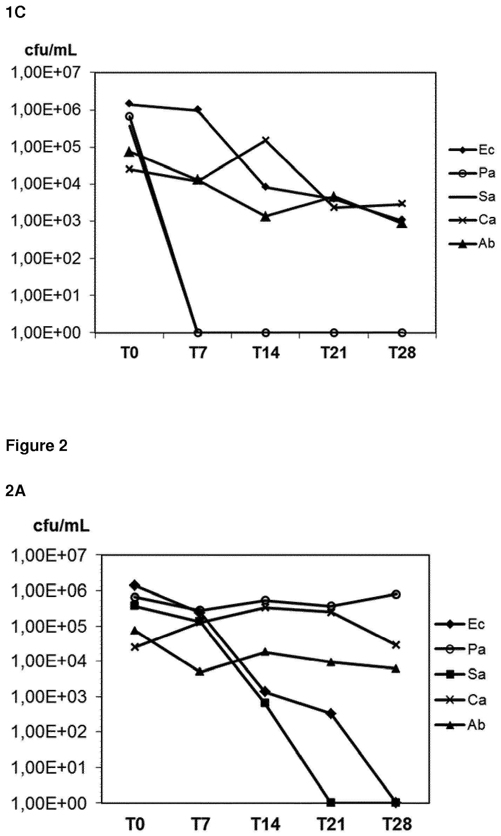

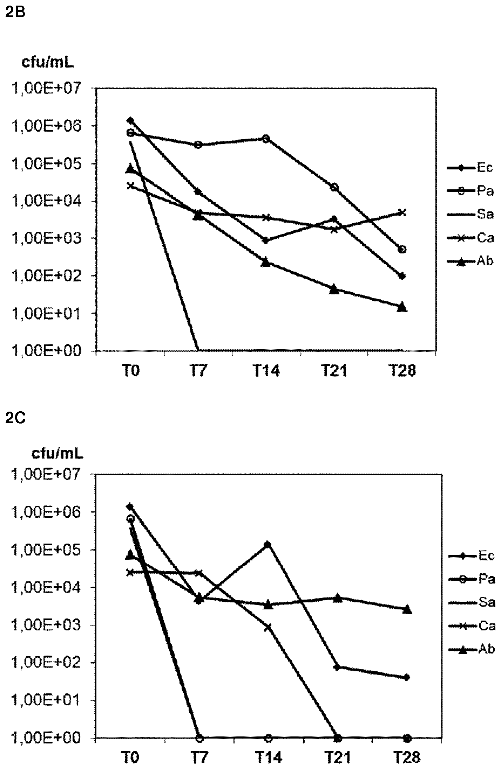

In order to confirm the activity against Gram-positive and Gram-negative bacteria, yeasts and moulds, conservation efficacy tests were performed according to DIN EN ISO 11930. The following classical contamination germs were examined: *Escherichia coli* (Ec in figures 1 and 2) ATCC®8739, *Pseudomonas aeruginosa* (Pa in figures 1 and 2) ATCC®9027, *Stapyhlococcus aureus* (Sa in figures 1 and 2) ATCC®6538, *Candida albicans* (Ca in figures 1 and 2) ATCC®10231 and *Aspergillus brasiliensis* (Ab in figures 1 and 2) ATCC®16404. Media composition, incubation conditions and dilution procedures were all conducted according to the guidelines. Levulinic acid alone does not fulfill the criteria according to the guidelines for an adequate conservation. Octane-1,2-diol alone does not fulfill the criteria according to the guidelines for an adequate conservation against C. *albicans* and P. *aeruginosa.* Perillic acid alone fulfills the criteria for an adequate conservation against E. *coli, S. aureus, C. albicans* and *A. brasiliensis.* The combination of perillic acid with levulinic acid and perillic acid with octane-1,2-diol using defined concentrations leads to blends fulfilling the criteria for an adequate conservation against all five test strains.

### Description of the figures

The results are shown for the combination of levulinic acid with perillic acid in figure 1 and for the combination of octane-1,2-diol with perillic acid in figure 2.

Data are presented as line graphs. The x-axis shows the different time points from the beginning of the experiment on day 0 (T0) up to the end of the experiment on day 28 (T28). The y-axis shows the colony forming units (cfu) of the tested contamination germs that were present per ml of cosmetic formulation. The tested contamination germs are identified by different symbols as indicated in the diagrams with the following abbreviations:
Ec: *Escherichia coli* (Gram-negative bacterium)
Pa: *Pseudomonas aeruginosa* (Gram-negative bacterium)
Sa: *Stapyhlococcus aureus* (Gram-positive bacterium)
Ca: *Candida albicans* (Yeast)
Ab: *Aspergillus brasiliensis* (Mould)
Figure 1A shows the results for levulinic acid alone (concentration range of C with 0.1-0.5% (w/v)). Figure 1B shows the results for perillic acid alone (concentration range of D with < 0.1% (w/v)). Figure 1C shows the results for the combination of levulinic acid and perillic acid (concentration range for both: D with < 0.1% (w/v)).
Figure 2A shows the results for octane-1,2-diol alone (concentration range of C with 0.1-0.5% (w/v)). Figure 2B shows the results for perillic acid alone (concentration range of D with < 0.1% (w/v)). Figure 2C shows the results for the combination of octane-1,2-diol and perillic acid (concentration range for both: D with < 0.1% (w/v)).

## Claims

1. A composition comprising
a. at least one perillic acid compound, and
b. at least one activity enhancing substance that enhances the activity of the perillic acid compound,
wherein the activity enhancing substance comprises at least two terminal oxygen residues and wherein the activity enhancing substance is selected from the group consisting of 1,2-diols, carboxylic acids and derivatives of carboxylic acids selected from the group consisting of salts of carboxylic acids, hydrolysable esters of carboxylic acids, and hydrolysable ethers of carboxylic acids.

2. Composition according to claim 1, wherein composition comprises the perillic acid compound in an amount of at least 0.00001 % (w/v).

3. Composition according to at least one of the preceding claims, wherein the perillic acid compound is used in the form of its R-enantiomer, its S-enantiomer or any mixture thereof, including racemic mixtures.

4. Composition according to at least one of the preceding claims, wherein the amount of water in the composition is at least 10% by weight of the composition.

5. Composition according to at least one of the preceding claims, wherein the amount (w/v) of activity enhancing substance in the composition is at least 10% of the amount (w/v) of perillic acid compound in the composition.

6. Composition according to at least one of the preceding claims, wherein the amount (w/v) of activity enhancing substance in the compositions is at most 1000 times as high as the amount (w/v) of perillic acid compound in the compositions.

7. Composition according to at least one of the preceding claims, wherein the terminal oxygen residues are bound to adjacent carbon atoms or to the same carbon atom within the activity enhancing substance to form a terminal oxygen group (TO-group).

8. Composition according to claim 7, wherein the TO-group is selected from the group consisting of the malic group, the glycol group and the carboxyl group.

9. Composition according to at least one of the preceding claims, wherein the activity enhancing substance is selected from the group consisting of hexane-1,2-diol, octane-1,2-diol, decane-1,2-diol, dodecane-1,2-diol, levulinic acid, p-anisic acid, propionic acid, pelargonic acid, malic acid, salicylic acid, sodium benzoate and potassium sorbate.

10. Composition according to at least one of the preceding claims, wherein the composition comprises the perillic acid compound in an amount of at least 0.00001 % (w/v) and at most 10% (w/v), wherein the amount (w/v) of activity enhancing substance in the composition is at least 10% of the amount of perillic acid compound in the composition and at most 1000 times as high as the amount of perillic acid compound in the composition.

11. Composition according to at least one of the preceding claims, wherein the composition is a cosmetic composition, a pharmaceutical composition, a nutritional composition, a plant-protection composition or an industrial composition.

12. Composition according to at least one of the preceding claims for use in a method of therapeutic treatment wherein the method includes treating a microbial infection.

13. Use of the composition according to at least one of claims 1 to 11 as an antimicrobial agent in a non-therapeutic method, or as a preservative.

14. Method of preparing a composition according to at least one of claims 1 to 11, including the step of
- preparing a mixture of a perillic acid compound and an activity enhancing substance.

## Patentansprüche

1. Zusammensetzung umfassend
a. mindestens eine Perillasäure-Verbindung, und
b. mindestens eine aktivitätsverstärkende Substanz, die die Aktivität der Perillasäure-Verbindung verstärkt,
wobei die aktivitätsverstärkende Substanz mindestens zwei terminale Sauerstoffreste umfasst und wobei die aktivitätsverstärkende Substanz aus der Gruppe ausgewählt ist,
welche aus 1,2-Diolen, Carbonsäuren und Derivaten von Carbonsäuren, ausgewählt aus der aus Salzen von Carbonsäuren, hydrolysierbaren Estern von Carbonsäuren und hydrolysierbaren Ethern von Carbonsäuren bestehenden Gruppe, besteht.

2. Zusammensetzung gemäß Anspruch 1, wobei eine Zusammensetzung die Perillasäure-Verbindung in einer Menge von mindestens 0,00001 % (w/v) umfasst.

3. Zusammensetzung gemäß mindestens einem der vorhergehenden Ansprüche, wobei die Perillasäure-Verbindung in der Form ihres *R*-Enantiomers, ihres *S*-Enantiomers oder von jedwedem Gemisch davon, einschließend racemische Gemische, verwendet wird.

4. Zusammensetzung gemäß mindestens einem der vorhergehenden Ansprüche, wobei die Menge an Wasser in der Zusammensetzung mindestens 10 Gew.-% der Zusammensetzung beträgt.

5. Zusammensetzung gemäß mindestens einem der vorhergehenden Ansprüche, wobei die Menge (w/v) an aktivitätsverstärkender Substanz in der Zusammensetzung mindestens 10 % der Menge (w/v) an Perillasäure-Verbindung in der Zusammensetzung beträgt.

6. Zusammensetzung gemäß mindestens einem der vorhergehenden Ansprüche, wobei die Menge (w/v) an aktivitätsverstärkender Substanz in den Zusammensetzungen höchstens 1000 Mal so hoch ist wie die Menge (w/v) an Perillasäure-Verbindung in den Zusammensetzungen.

7. Zusammensetzung gemäß mindestens einem der vorhergehenden Ansprüche, wobei die terminalen Sauerstoffreste an benachbarte Kohlenstoffatome oder an dasselbe Kohlenstoffatom in der aktivitätsverstärkenden Substanz, wobei ein terminaler Sauerstoffrest (TO-Rest) gebildet wird, gebunden sind.

8. Zusammensetzung gemäß Anspruch 7, wobei der TO-Rest aus der Gruppe, welche aus der Äpfel(säure)gruppe, der Glycolgruppe und der Carboxylgruppe besteht, ausgewählt ist.

9. Zusammensetzung gemäß mindestens einem der vorhergehenden Ansprüche, wobei die aktivitätsverstärkende Substanz aus der Gruppe, welche aus Hexan-1,2-diol, Octan-1,2-diol, Decan-1,2-diol, Dodecan-1,2-diol, Lävulinsäure, *p*-Anissäure, Propionsäure, Pelargonsäure, Äpfelsäure, Salicylsäure, Natriumbenzoat und Kaliumsorbat besteht, ausgewählt ist.

10. Zusammensetzung gemäß mindestens einem der vorhergehenden Ansprüche, wobei die Zusammensetzung die Perillasäure-Verbindung in einer Menge von mindestens 0,00001 % (w/v) und höchstens 10 % (w/v) umfasst, wobei die Menge (w/v) an aktivitätsverstärkender Substanz in der Zusammensetzung mindestens 10 % der Menge an Perillasäure-Verbindung in der Zusammensetzung beträgt und höchstens 1000 Mal so hoch ist wie die Menge an Perillasäure-Verbindung in den Zusammensetzung.

11. Zusammensetzung gemäß mindestens einem der vorhergehenden Ansprüche, wobei die Zusammensetzung eine kosmetische Zusammensetzung, eine pharmazeutische Zusammensetzung, eine Nahrungszusammensetzung, eine Pflanzenschutzzusammensetzung oder eine industrielle Zusammensetzung ist.

12. Zusammensetzung gemäß mindestens einem der vorhergehenden Ansprüche zur Verwendung in einem Verfahren zur therapeutischen Behandlung, wobei das Verfahren Behandeln einer mikrobiellen Infektion einschließt.

13. Verwendung der Zusammensetzung gemäß mindestens einem der Ansprüche 1 bis 11 als ein antimikrobielles Mittel in einem nicht-therapeutischen Verfahren, oder als ein Konservierungsmittel.

14. Verfahren zum Herstellen einer Zusammensetzung gemäß mindestens einem der Ansprüche 1 bis 11, einschließend den Schritt von
- Herstellen eines Gemisches von einer Perillasäure-Verbindung und einer aktivitätsverstärkenden Substanz.

## Revendications

1. Composition comprenant
a. au moins un composé d'acide périllique, et
b. au moins une substance de renforcement d'activité qui renforce l'activité du composé d'acide périllique,
dans laquelle la substance de renforcement d'activité comprend au moins deux résidus oxygène terminaux et dans laquelle la substance de renforcement d'activité est choisie dans le groupe consistant en les 1,2-diols, les acides carboxyliques et les dérivés d'acides carboxyliques choisis dans le groupe consistant en les sels d'acides carboxyliques, les esters hydrolysables d'acides carboxyliques et les éthers hydrolysables d'acides carboxyliques.

2. Composition selon la revendication 1, dans laquelle la composition comprend le composé d'acide périllique selon une quantité d'au moins 0,00001 % (en poids/volume).

3. Composition selon au moins une des revendications précédentes, dans laquelle le composé d'acide périllique est utilisé sous la forme de son *R-*énantiomère, de son *S*-énantiomère ou de tout mélange de ceux-ci, y compris les mélanges racémiques.

4. Composition selon au moins une des revendications précédentes, dans laquelle la quantité d'eau dans la composition est d'au moins 10 % en poids de la composition.

5. Composition selon au moins une des revendications précédentes, dans laquelle la quantité (en poids/volume) de substance de renforcement d'activité dans la composition est d'au moins 10 % de la quantité (en poids/volume) de composé d'acide périllique dans la composition.

6. Composition selon au moins une des revendications précédentes, dans laquelle la quantité (en poids/volume) de substance de renforcement d'activité dans les compositions est au plus 1000 fois supérieure à la quantité (en poids/volume) de composé d'acide périllique dans les compositions.

7. Composition selon au moins une des revendications précédentes, dans laquelle les résidus oxygène terminaux sont liés à des atomes de carbone adjacents ou au même atome de carbone dans la substance de renforcement d'activité pour former un groupe oxygène terminal (groupe OT).

8. Composition selon la revendication 7, dans laquelle le groupe OT est choisi dans le groupe constitué par le groupe malique, le groupe glycol et le groupe carboxyle.

9. Composition selon au moins une des revendications précédentes, dans laquelle la substance de renforcement d'activité est choisie dans le groupe constitué par l'hexane-1,2-diol, l'octane-1,2-diol, le décane-1,2-diol, le dodécane-1,2-diol, l'acide lévulinique, l'acide *p*-anisique, l'acide propionique, l'acide pélargonique, l'acide malique, l'acide salicylique, le benzoate de sodium et le sorbate de potassium.

10. Composition selon au moins une des revendications précédentes, dans laquelle la composition comprend le composé d'acide périllique selon une quantité d'au moins 0,00001 % (en poids/volume) et d'au plus 10 % (en poids/volume), dans laquelle la quantité (en poids/volume) de substance de renforcement d'activité dans la composition est d'au moins 10 % de la quantité de composé d'acide périllique dans la composition et au plus 1000 fois supérieur à la quantité de composé d'acide périllique dans la composition.

11. Composition selon au moins une des revendications précédentes, dans laquelle la composition est une composition cosmétique, une composition pharmaceutique, une composition nutritionnelle, une composition phytoprotectrice ou une composition industrielle.

12. Composition selon au moins une des revendications précédentes destinée à être utilisée dans un procédé de traitement thérapeutique, dans laquelle le procédé comprend le traitement d'une infection microbienne.

13. Utilisation de la composition selon au moins une des revendications 1 à 11 en tant qu'agent antimicrobien dans un procédé non-thérapeutique ou en tant que conservateur.

14. Procédé de préparation d'une composition selon l'une quelconque des revendications 1 à 11, comprenant l'étape de
- préparer un mélange d'un composé d'acide périllique et d'une substance de renforcement d'activité.
